# EUROPÄISCHE PATENTSCHRIFT

(11) **EP 3 150 117 B1**
(45) Veröffentlichungstag und Bekanntmachung des Hinweises auf die Patenterteilung: **06.03.2019**
(21) Anmeldenummer: 16188459.8
(22) Anmeldetag: 13.09.2016
(51) Int. Cl.: A61B 5/0215

(54) **IMPLANTIERBARE DRUCKSENSORVORRICHTUNG**
IMPLANTABLE PRESSURE SENSOR
DISPOSITIF DE CAPTEUR DE PRESSION IMPLANTABLE

(30) Priorität: 01.10.2015 DE 102015116648
(43) Veröffentlichungstag der Anmeldung: 05.04.2017
(73) Patentinhaber: BIOTRONIK SE & Co. KG, 12359 Berlin (DE)
(72) Erfinder: Bitzer, Andreas, 8032 Zürich (CH); Pfenniger, Alois, 2502 Biel (CH); Ebert, Henning, 12437 Berlin (DE); van Ooyen, Andrè, 10717 Berlin (DE); Elsner, Joachim, 14059 Berlin (DE)
(74) Vertreter: Galander, Marcus

(56) Entgegenhaltungen:
- WO-A1-2013/003754
- WO-A2-2014/197101
- DE-A1-102011 054 732
- US-A- 4 593 703

## Beschreibung

Die Erfindung betrifft eine implantierbare Drucksensoreinrichtung, insbesondere eine Miniaturdrucksensoreinrichtung, die in einen tierischen oder menschlichen Körper implantierbar ist.

Drucksensorvorrichtungen einschließlich mikroelektromechanische Systeme (MEMS) und dazugehörige Chipsysteme sind in der Technik bekannt. Beispielsweise werden Drucksensorvorrichtungen mit MEMS-Chips für elektrische Implantate zur Verbesserung der Herzfunktion eines Patienten eingesetzt. Für derartige Zwecke einsetzbare Chipsysteme müssen einerseits hinreichend präzise Messergebnisse liefern, andererseits sehr kleine Abmessungen aufweisen, sodass sie problemlos implantierbar sind und die physiologische Aktivität des Organismus nicht einschränken, beispielsweise für Blutdruckmessungen in der Pulmonararterie.

Für den Einsatz von MEMS-Chips in einem reaktiven Medium, wie z.B. in Blut, muss der Chip in der Regel geschützt werden. Dazu werden sie typischerweise in einer inkompressiblen und inerten Flüssigkeit eingebettet und in einem Gehäuse hermetisch gegen das reaktive Medium abgeschlossen. Die Flüssigkeit (z.B. Öl) dient dabei als Druckübertragungsmedium, sodass der äußere Druck über das Gehäuse zum MEMS-Chip geleitet werden kann. Als Gehäusematerial ist beispielsweise Titan durch seine Langzeitstabilität und hohe Biokompatibilität geeignet.

Im Blutkreislauf eines Patienten können gewöhnlich Temperaturänderungen von mehreren Grad Celsius auftreten, wodurch es zu Volumenänderungen des Druckübertragungsmediums innerhalb des Gehäuses kommt. Ein weiteres Problem ist die Temperänderung während einer Sterilisation, beispielsweise mittels Ethylenoxid, wobei Temperaturdifferenzen von ca. 30 Grad Celsius vorkommen. Die daraus resultierende Volumen- und Druckzunahme kann bei einem konventionellen Drucksensorgehäuse die Membranen oder den MEMS-Chips beschädigen. Da das Gehäuse üblicherweise eine geringe Nachgiebigkeit bzw. Elastizität aufweist, führen die besagten Volumenänderungen des Druckübertragungsmediums zu hohen Druckschwankungen innerhalb des Gehäuses. Dieser Umstand ist zum einen durch die Materialeigenschaften des Gehäuses, zum anderen durch die zur Gehäusegröße verhältnismäßig dicken Gehäusewände bedingt. Resultierend werden die Druckmesswerte des MEMS-Chips verfälscht, da die zu messenden Druckwerte durch temperaturbedingte Druckschwankungen im Inneren des Gehäuses überlagert werden.

In US 8,573,062 B2 ist für eine MEMS-Chip Sensoranordnung die Verwendung einer Druckübertragungsmembran beschrieben, welche ein Fenster bedeckt, das in die Seitenwand des Gehäuses der Sensoranordnung eingelassen ist. In US 8 142 362 B2 ist eine an der Stirnseite des Gehäuses angeordnete Druckübertragungsmembran beschrieben.

Das Dokument WO 2013 / 003754 A1 offenbart einen implantierbaren Sensor mit dünnen Seitenwänden.

Das Dokument DE 10 2011 054 732 A1 offenbart ein Implantat zum Messen des Drucks in einer Körperflüssigkeit. Das Implantat weist ein Gehäuse auf, in welchem ein Drucksensor angeordnet ist. Das Gehäuse hat eine erste Öffnung, die mit einer ersten Membran verschlossen ist. Des Weiteren weist das Gehäuse eine zweite Öffnung auf, welche mit einer zweiten Membran verschlossen ist.

Das Dokument US 4,593,703 A offenbart eine implantierbare Vorrichtung zum Messen eines Drucks.

Das Dokument WO 2014 / 197101 A2 offenbart ein weiteres Implantat zur Druckmessung.

Bisherige Lösungen aus dem Stand der Technik zur Reduktion der Temperaturabhängigkeit von MEMS-Chip Sensorsystemen können die Verfälschung der Druckmesswerte nicht hinreichend reduzieren, sodass sie gegenüber den zu messenden Druckwerten in der Körperumgebung vernachlässigbar würden. Weitere Probleme sind u.a. ein relativ hoher Fertigungsaufwand und eine geringe Robustheit des Gehäuses gegenüber Materialspannungen und Kräfte auf das Gehäuse, die ebenfalls durch die Volumenänderung des Druckübertragungsmediums auftreten. So können fertigungsbedingte Fügestellen des Gehäuses durch Zugspannungen aufreißen, wenn das Innenvolumen des Gehäuses expandiert. Wird beispielsweise eine einzelne flache Druckübertragungsmembran für das Gehäuse des Sensorsystems verwendet, tritt bei Volumenänderungen im Inneren des Gehäuses longitudinale Dehnung des Gehäusematerials auf, die Folge sind hohe Kräfte auf Schweißnähte und es entstehen hohe Druckwiderstände.

Es ist daher eine Aufgabe der Erfindung, eine implantierbare Drucksensorvorrichtung bereitzustellen, die überwiegend unempfindlich gegenüber Temperaturänderungen in der Betriebsatmosphäre und die damit auftretenden Materialspannungen ist.

Die Aufgabe wird durch die Merkmale des unabhängigen Anspruchs 1 gelöst. Die weiteren Ansprüche beschreiben vorteilhafte Ausführungsformen der Erfindung.

Mit dem Einsatz von zwei Druckübertragungsmembranen werden für das Gehäuse der implantierbaren Drucksensorvorrichtung zwei nicht zusammenhängende Flächen mit größerer Nachgiebigkeit bzw. Elastizität geschaffen, sodass der Druck im Inneren des Gehäuses bei Temperaturschwankungen hinreichend reduziert wird. Indem die zwei Druckübertragungsmembranen nicht in einer Ebene angeordnet sind, können auch die durch Volumenänderung im Inneren des Gehäuses entstehenden Spannungen und Kräfte auf das Gehäusematerial sich mindestens teilweise kompensieren, wodurch die Gehäuseanordnung an Robustheit gewinnt.

In einer vorteilhaften Ausgestaltung bilden die Druckübertragungsmembranen einen Bestandteil von mindestens zwei unterschiedlichen Außenwänden der Gehäuseanordnung. Vorzugsweise liegen sich dabei die zwei unterschiedlichen Außenwände gegenüber. Dabei ist die Gehäuseanordnung bevorzugt zumindest teilweise als Quader, Zylinder, Kugel oder Ellipsoid gestaltet.

In dem jene Außenwände der Gehäuseanordnung, deren einer Bestandteil durch eine Druckmembran gebildet wird, sich gegenüberliegend angeordnet sind, wird eine gleichmäßigere Kräfte- und Spannungsverteilung auf das Gehäuse im Fall von Volumenänderungen im Inneren des Gehäuses erzielt. Genauer gesagt entstehen infolgedessen kleinere Spannungsmaxima und es wird erwirkt, dass sich ein besonders großer Teil der Kräfte und Spannungen kompensiert, sodass für derartige Einwirkungen empfindliche Bereiche des Gehäuses entlastet werden. Auf diese Weise wird eine besonders vorteilhafte Konfiguration hinsichtlich der Robustheit des Gehäuses gegenüber Temperaturschwankungen geschaffen. Im Allgemeinen erwirkt ein planarer, spiegelsymmetrischer Aufbau der Gehäuseanordnung weitere Vorteile für eine gemäßigtere Spannungsbelastung der gesamten Gehäuseanordnung. Unter einem spiegelsymmetrischen Aufbau wird im Rahmen der Anmeldung eine Gehäuseanordnung verstanden, deren äußere Geometrie symmetrisch zu mindestens einer Schnittebene ist. So stellt eine besonders vorteilhafte äußere Geometrie der Gehäuseanordnung ein flacher Quader dar, bei dem zwei flache, rechteckige Druckübertragungsmembranen sich gegenüberliegend auf zwei Außenwänden angeordnet sind. Die Druckübertragungsmembranen können dabei in zwei entsprechende Fenster integriert sein, die in das Gehäuse eingelassen sind. Beispielsweise können die Druckübertragungsmembranen die Fenster bedecken und an den Randbereichen mit dem Gehäuse fixiert sein oder in die Fenster eingespannt sein. Eine weitere Ausgestaltung sieht die Verwendung von runden Druckübertragungsmembranen vor. Auch können die Druckübertragungsmembranen vorgewölbt und/oder mit einem Wellenprofil versehen sein. Eine günstige Ausgestaltung eines Wellenprofils stellt eine gewellte Innenfläche der Druckübertragungsmembran dar. Auf diese Weise können die Biegeeigenschaften des Gehäuses verbessert werden.

Gemäß einer weiteren vorteilhaften Ausführung weist mindestens eine Außenwand der Gehäuseanordnung einen reversibel deformierbaren Bereich auf.

Mit reversibel deformierbaren Bereichen der Außenwand wird die Nachgiebigkeit bzw. Elastizität der Gehäuseanordnung vergrößert, da ein solcher Bereich durch Volumenänderungen im Inneren des Gehäuses entstehende Spannungen und Kräfte kompensiert und somit nicht an die wenig elastischen Bereiche der Gehäuseanordnung weiterleitet. Beispielsweise kann ein solcher reversibel deformierbarer Bereich in der Zone zwischen der elastischen Druckübertragungsmembran und weniger elastischen Außenwandarealen der Gehäuseanordnung eingesetzt werden, sodass diese Zonen bei Beanspruchung durch Spannungen entlastet werden.

Bevorzugt weist die Gehäuseanordnung mindestens eine Aussparung auf, wobei die Aussparung ausgelegt ist, durch Wärmeausdehnungseffekte hervorgerufene Verformungen des Innenvolumens und/oder von mindestens einer Druckübertragungsmembran und/oder von mindestens einer Außenwand aufzunehmen. Vorzugsweise ist die Aussparung im Innenvolumen der Gehäuseanordnung angeordnet und kann beispielsweise als Fuge, Loch, Schlitz, Spalte, Kerbe, Nut oder Einschnitt gestaltet sein.

Vorzugsweise ist die Aussparung zwischen dem MEMS-Chip und der Gehäuseanordnung angeordnet, sodass sie den direkten Kontakt des MEMS-Chips und der Gehäuseanordnung mindestens teilweise unterbindet, sodass der MEMS-Chip effektiv gegen die Druckmessungen verfälschende Einflüsse geschützt ist. Bevorzugt ist die Aussparung in Arealen angeordnet, in denen größere Deformationen durch Wärmeausdehnungseffekte der Drucksensorvorrichtung zu erwarten sind, wie zum Beispiel angrenzend am reversibel deformierbaren Bereich der Außenwand der Gehäuseanordnung.

Ebenfalls kann der deformierbare Bereich einer Außenwand durch geeignete Anordnung der Aussparung erzeugt werden, wie z.B. durch Platzierung einer Fuge entlang eines Bereichs der Außenwand, sodass sich dieser Bereich bei Temperaturänderungen freier verformen und zurückverformen kann. Der reversibel deformierbare Bereich einer Außenwand kann auch erzeugt werden, in dem die Dicke der Außenwand an geeigneter Stelle durch Abtragung des Materials reduziert wird.

Gemäß einer weiteren bevorzugten Ausgestaltung grenzt mindestens eine Druckübertragungsmembran an die Aussparung oder ist an diese gekoppelt.

In einer weiteren vorteilhaften Ausführung grenzt mindestens eine Außenwand an die Aussparung oder ist an diese gekoppelt.

Besonders vorteilhafte Eigenschaften für die Gehäuseanordnung werden erreicht, wenn die Druckübertragungsmembranen über einen reversibel deformierbaren Bereich einer Außenwand an das Gehäuse gekoppelt sind. Im Inneren des Gehäuses, in dem sich der MEMS-Chip befindet, wird die Aussparung so angeordnet, dass sie den direkten Kontakt des MEMS-Chips mit der Gehäuseanordnung mindestens teilweise unterbindet.

Durch die kombinierte Anordnung und Verbindung von Elementen mit hoher Elastizität und mindestens einer Aussparung wird eine Gehäuseanordnung konstruiert, die durch Wärmeausdehnung entstehende Drücke im Inneren des Gehäuses sowie Spannungen und Kräfte, die auf das Gehäuse wirken, effektiv kompensiert. Möglich sind verschiedene Anordnungen und Variationen dieser Elemente für die Gehäuseanordnung. Dabei können die verschiedenen Elemente und Bestandteile der Gehäuseanordnung diese aus unterschiedlichen, für die Anwendung zweckmäßigen Materialien gefertigt sein.

Gemäß einer weiteren vorteilhaften Ausführung weist die Gehäuseanordnung ein Auflagemodul im Innenvolumen auf, wobei das Auflagemodul an die Aussparung grenzt oder gekoppelt ist. Vorzugsweise ist das Auflagemodul an mindestens eine Außenwand gekoppelt und ein mikroelektromechanischer Drucksensorchip im Innenvolumen der Gehäuseanordnung angeordnet oder mit dem Innenvolumen gekoppelt, wobei der Drucksensorchip mit dem Auflagemodul verbunden ist.

Bevorzugt dient das Auflagemodul zur Lagerung des MEMS-Chips. Mittels der Aussparung werden die Bereiche direkter Materialverbindung zwischen Auflagemodul und der restlichen Gehäuseanordnung, insbesondere den Außenwänden des Gehäuses, verringert. Die Auflagefläche ist dabei so dimensioniert, dass der aufgelagerte MEMS-Chip ebenfalls durch die Aussparung wenigstens teilweise vom Rest der Gehäusevorrichtung entkoppelt ist. Vorzugsweise ist der MEMS-Chip durch die Konstruktion innerhalb der Gehäuseanordnung auf dem Auflagemodul wie auf einem Steg gelagert und steht in möglichst geringfügigem Kontakt zum Rest der Gehäuseanordnung.

Vorzugsweise ist das Innenvolumen der Gehäuseanordnung mit einem Druckübertragungsmedium befüllbar, wie beispielsweise Öl. In einer weiteren vorteilhaften Ausführung weisen die Außenwände der Gehäuseanordnung mindestens einen Durchbruch zum Innenvolumen der Gehäuseanordnung auf.

Ein Durchbruch stellt beispielsweise eine Durchführung zur Kontaktierung des MEMS-Chips mit anderen elektronischen Komponenten dar, wie z.B. einer Batterie oder einer Antenne. Auch kann ein Durchbruch ein Loch darstellen, über welches das Innere der Gehäuseanordnung mit dem Druckübertragungsmedium befüllt werden kann. Der Durchbruch ist dabei wahlweise verschließbar.

Vorteilhafterweise ist die Gehäuseanordnung zumindest teilweise aus Titan gefertigt. Möglich sind jedoch auch andere für diesen Zweck geeignete Materialien. Neben der bevorzugten Ausgestaltung der Gehäuseanordnung in Quaderform kann auch eine geometrische Form mit einem dreieckigen, elliptischen Querschnitt oder dergleichen angewendet werden.

Bevorzugt ist die Gehäuseanordnung konfiguriert, sodass Messgenauigkeiten von ca. 2 mbar mit dem MEMS-Chip erzielt werden können.

Gemäß einer vorteilhaften Ausführungsform beträgt die Dicke der Druckübertragungsmembran nicht mehr als 30 µm, vorzugsweise nicht mehr als 10 µm. Dies bietet eine ausreichende Flexibilität und zugleich Stabilität für das Gehäuse der Drucksensorvorrichtung.

Generell kann die Gehäuseanordnung auch in anderen Bereichen anwendbar sein, in denen Volumenänderungen zu Gunsten der Funktionalität elektronischer Bauteile kompensiert werden müssen, wie beispielsweise im Bereich von Volumenänderungen durch chemische Prozesse in Li-Ionen-Batterien.

Zusammen mit den oben erwähnten und anderen Aufgaben und Vorteilen kann die Erfindung am besten anhand der folgenden detaillierten Beschreibung der Ausführungsbeispiele verstanden werden, ohne jedoch auf diese beschränkt zu sein.

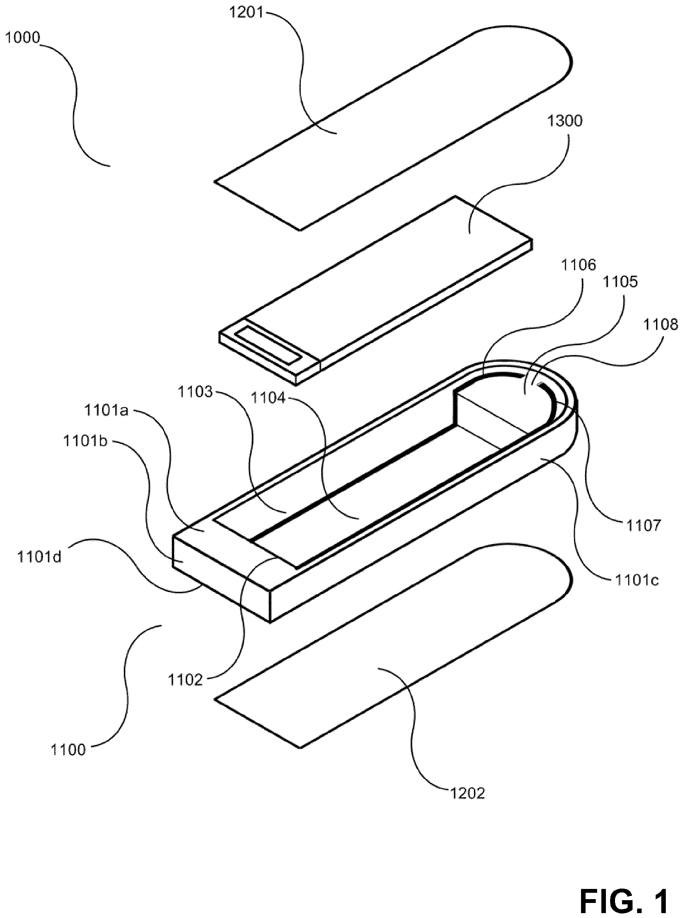

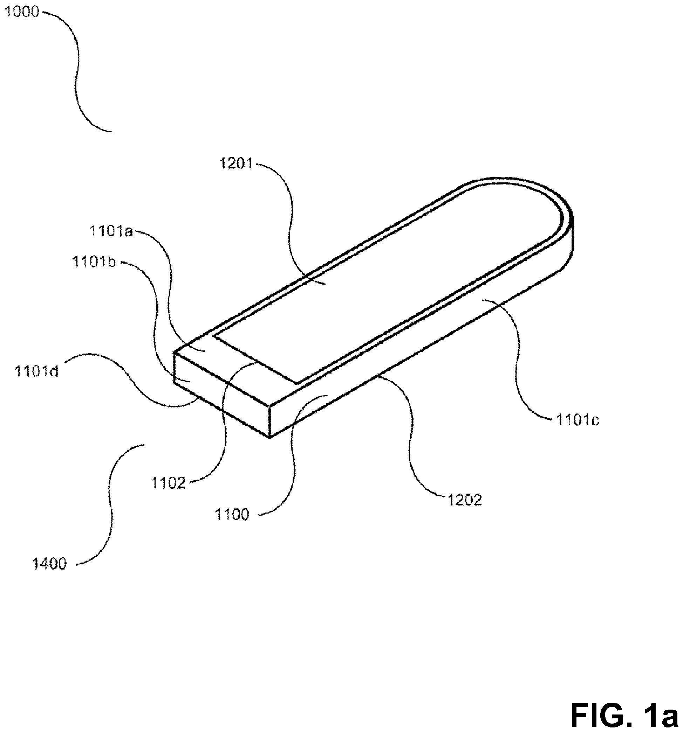

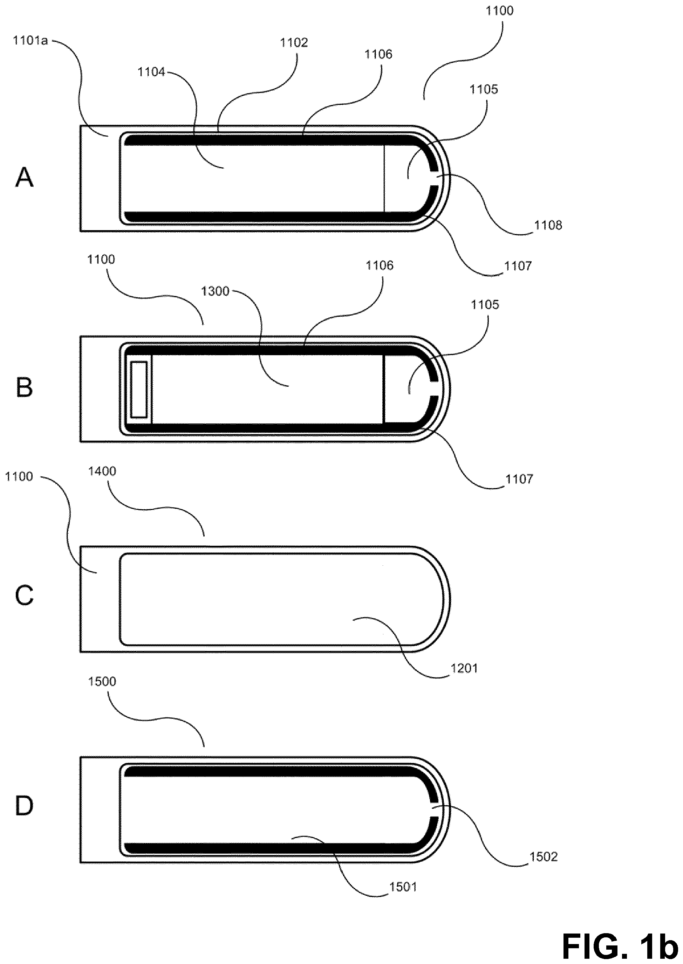

- Figur 1:: Explosionsansicht einer implantierbaren Drucksensorvorrichtung gemäß einer Ausführungsform
- Figur 1 a:: Perspektivische Ansicht der implantierbaren Drucksensorvorrichtung gemäß einer Ausführungsform nach Figur 1 in fertig montierter Form
- Figur 1b:: Verschiedene Draufsichten der implantierbaren Drucksensorvorrichtung gemäß einer Ausführungsform nach Figur 1
- Figur 2:: Explosionsansicht der implantierbaren Drucksensorvorrichtung gemäß einer weiteren Ausführungsform
- Figur 2a:: Perspektivische Ansicht der implantierbaren Drucksensorvorrichtung gemäß einer weiteren Ausführungsform nach Figur 2
- Figur 2b:: Verschiedene Draufsichten der implantierbaren Drucksensorvorrichtung gemäß einer weiteren Ausführungsform nach Figur 2
- Figur 3:: Gehäuse einer Drucksensorvorrichtung mit Durchbrüchen gemäß weiterer Ausführungsformen
- Figur 4:: Diagramm mit Druck-Volumen-Kurven für Gehäusetopologien aus dem Stand der Technik und der Gehäuseanordnung
- Figur 5:: Ausführungsform der implantierbaren Drucksensorvorrichtung mit angeschlossenem Batteriemodul
- Figur 6a:: Ausführungsform der implantierbaren Drucksensorvorrichtung mit angeschlossenem Batteriemodul und Befestigungsvorrichtung
- Figur 6b:: Ausführungsform der implantierbaren Drucksensorvorrichtung mit angeschlossenem Batteriemodul und weiterer Befestigungsvorrichtung

In den Zeichnungen sind gleiche Elemente mit gleichen Bezugszeichen bezeichnet. Die Zeichnungen sind lediglich schematische Darstellungen und stellen keine spezifischen Parameter der Erfindung dar. Ferner sollen die Zeichnungen nur typische Ausführungsformen illustrieren und daher nicht als Einschränkung des Umfangs der Erfindung angesehen werden. Die in der Beschreibung angegebenen Abmessungen und die Werte sind nur als Beispiel zu verstehen und können in anderen Ausführungsformen unterschiedlich sein.

Figuren 1, 1a und 1b zeigen eine beispielhafte Ausführungsform einer implantierbaren Drucksensorvorrichtung 1000 in verschiedenen Perspektiven.

Figur 1 zeigt die Ausführungsform der Drucksensorvorrichtung 1000 in Explosionsansicht. Die Drucksensorvorrichtung 1000 besitzt eine längliche rechteckige Form und einem abgerundeten Ende. Eine derartige Geometrie bietet Vorteile bezüglich des Herstellungsaufwands sowie einer unkomplizierteren Handhabung für die Implantation. Dabei besteht die Drucksensorvorrichtung 1000 im Wesentlichen aus einem Gehäusegerüst 1100, zwei Druckübertragungsmembranen 1201 und 1202 sowie einem MEMS-Chip 1300. Das Gehäusegerüst umfasst ein Innenvolumen 1103, in das der MEMS-Chip 1300 auf ein Auflagemodul 1104 gelagert und befestigt wird. Außerdem umfasst das Gehäusegerüst verschiedene Außenwände 1101a - 1101d, wobei Außenwand 1101a ein Fenster 1102 besitzt. Außenwand 1101d auf der Unterseite des Gehäusegerüsts gleicht 1101a und besitzt ebenfalls ein Fenster 1102' (nicht sichtbar in Figur 1). Des Weiteren kann das Auflagemodul 1104 einen Vorsprung 1105 umfassen, welcher beispielsweise zur Stabilisierung des MEMS-Chips genutzt werden kann. Aussparungen in Form von Fugen 1106 und 1107 verlaufen entlang der Längsseiten des Auflagemoduls 1104 und entkoppeln ebendort das Auflagemodul 1104 von der Außenwand 1101c. Verbindungsstelle 1108 zwischen den beiden Enden der Fugen 1106 und 1107 stellt eine Materialverbindung zwischen Auflagemodul 1104 und dem Gehäusegerüst her.

Figur 1a zeigt die Ausführungsform der Drucksensorvorrichtung 1000 in fertig montierter Form. Das Gehäuse 1400 wird gebildet durch das Gehäusegerüst 1100 sowie den Druckübertragungsmembranen 1201 und 1202. Das Fenster 1102 und 1102' in der Außenwand 1101d (nicht zu sehen in Figur 1a) werden von den Druckübertragungsmembranen 1201 und 1202 bedeckt und vorzugsweise mit den Außenwänden 1101a und 1101d fest verbunden. So bilden die Druckübertragungsmembranen einen Bestandteil der Außenwände des Gehäuses 1400. Außenwände 1101a und 1101d sind vorzugsweise gleich gestaltet und daher entspricht die Ansicht der Unterseite des Gehäuses 1400 ebenfalls der Darstellung in Figur 1a. Auch möglich ist eine Ausführungsform, bei der die Fenster 1102 und 1102' versetzt voneinander angeordnet sind, wodurch dann die Außenwände 1101a und 1101d sich durch die Position des Fensters unterscheiden.

Figur 1b zeigt Draufsichten A, B, C und D der Ausführungsform der Drucksensorvorrichtung 1000 und deren Bestandteile. A in Figur 1b zeigt das Gehäusegerüst 1100 ohne MEMS-Druckchip und Druckübertragungsmembranen. Zu sehen ist die Außenwand 1101a des Gehäusegerüsts mit Fenster 1102, das Auflagemodul 1104 mit Vorsprung 1105 und Verbindungsstelle 1108 sowie die Aussparungen in Form von Fugen 1106 und 1107. B in Figur 1b zeigt das Gehäusegerüst 1100 mit eingelegtem MEMS-Chip 1300. Dabei ist der MEMS-Chip 1300 so auf dem Auflagemodul gelagert, dass er durch die Fugen 1106 und 1107 an zwei Längsseiten vom Gehäusegerüst 1100 entkoppelt ist. Vorsprung 1105 kann dazu genutzt werden, um MEMS-Chip 1300 zu stabilisieren. C in Figur 1b zeigt das Gehäuse 1400 der Drucksensorvorrichtung, welches gebildet wird durch das Gehäusegerüst 1100 und den Druckübertragungsmembranen 1201 und 1202 (auf der Unterseite). D in Figur 1b zeigt eine weitere mögliche Ausführungsform des Gehäusegerüsts ähnlich wie 1100, mit dem Unterschied dass ein Vorsprung weggelassen wird, sodass das Auflagemodul 1501 flach ausgelegt ist und direkt an Verbindungsstelle 1502 anschließt.

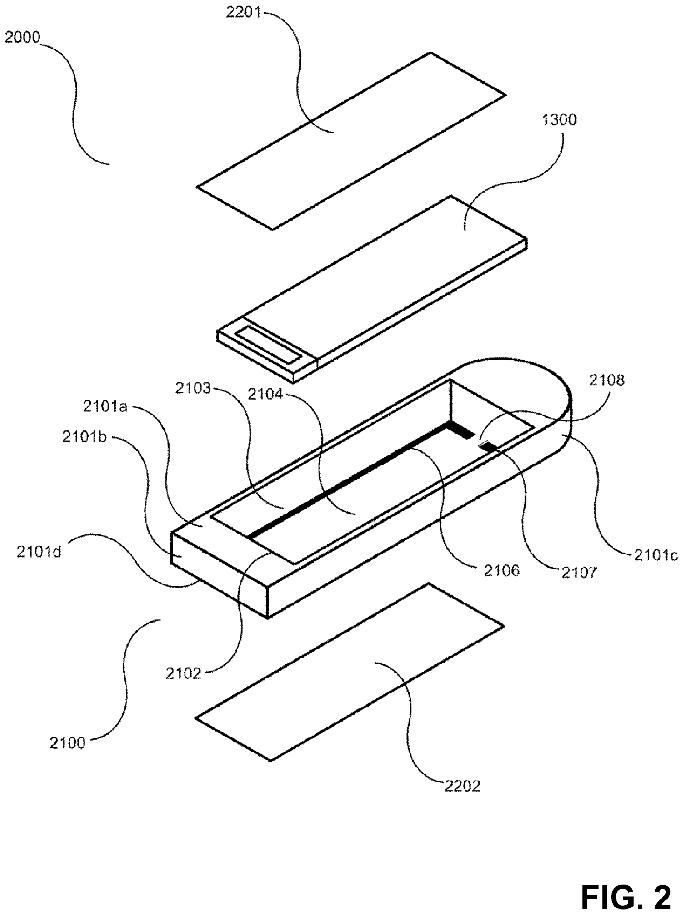

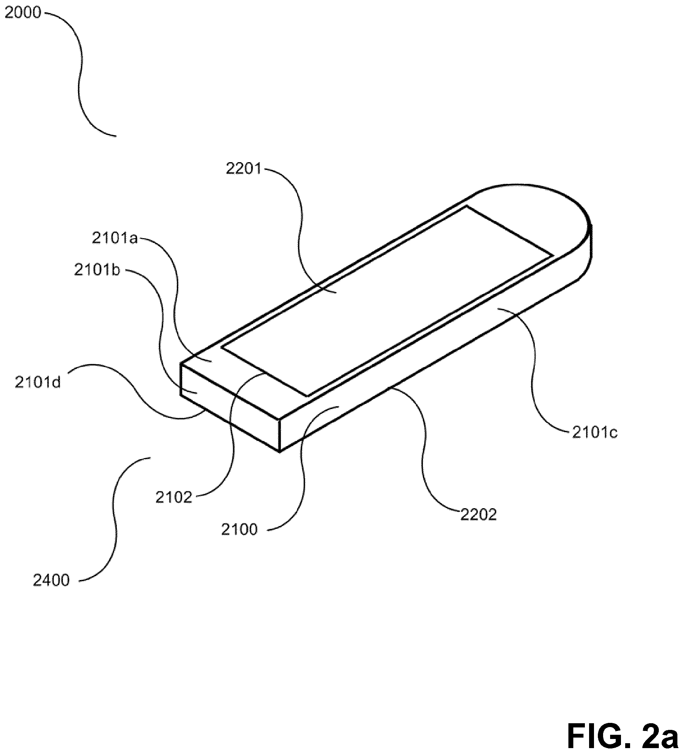

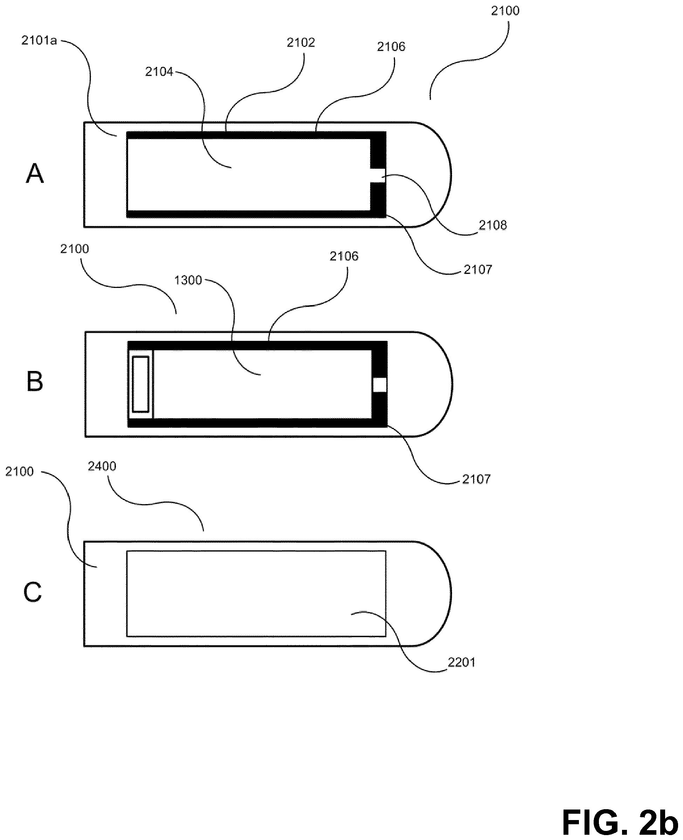

Figuren 2, 2a und 2b zeigen eine weitere beispielhafte Ausführungsform einer implantierbaren Drucksensorvorrichtung 2000 in verschiedenen Perspektiven.

Figur 2 zeigt die Ausführungsform der Drucksensorvorrichtung 2000 in Explosionsansicht. Die Drucksensorvorrichtung 2000 besitzt eine längliche rechteckige Form und einem abgerundeten Ende. Ähnlich zur Drucksensorvorrichtung 1000 besteht Drucksensorvorrichtung 2000 im Wesentlichen aus einem Gehäusegerüst 2100, zwei Druckübertragungsmembranen 2201 und 2202 sowie einem MEMS-Chip 1300. Das Gehäusegerüst umfasst ein Innenvolumen 2103, in das der MEMS-Chip 1300 auf ein Auflagemodul 2104 gelagert und befestigt wird. Außerdem umfasst das Gehäusegerüst verschiedene Außenwände 2101a - 2101d, wobei Außenwand 2101a ein Fenster 2102 besitzt. Außenwand 2101d auf der Unterseite des Gehäusegerüsts gleicht 2101a und besitzt ebenfalls ein Fenster 2102' (nicht sichtbar in Figur 2). Aussparungen in Form von Fugen 2106 und 2107 verlaufen entlang der Längsseiten des Auflagemoduls 2104 und entkoppeln ebendort das Auflagemodul 2104 von der Außenwand 2101c. Verbindungsstelle 2108 zwischen den beiden Enden der Fugen 2106 und 2107 stellt eine Materialverbindung zwischen Auflagemodul 2104 und dem Gehäusegerüst her. Im Vergleich zu Drucksensorvorrichtung 1000 ist das Innenvolumen 2013 der Drucksensorvorrichtung 2000 quaderförmig und besitzt, ungleich dem Innenvolumen 1013, kein abgerundetes Ende. Die in die Außenwände 2101a und 2011d eingelassenen Fenster 2102 und 2102' sowie die Druckübertragungsmembranen 2201 und 2202 sind rechteckig und besitzen kein abgerundetes Ende, wodurch der Fertigungsaufwand für die entsprechenden Komponenten reduziert werden kann.

Figur 2a zeigt die Ausführungsform der Drucksensorvorrichtung 2000 in fertig montierter Form. Das Gehäuse 2400 wird gebildet durch das Gehäusegerüst 2100 sowie den Druckübertragungsmembranen 2201 und 2202. Das Fenster 2102 und 2102' in der Außenwand 2101d (nicht zu sehen in Figur 2a) werden von den Druckübertragungsmembranen 2201 und 2202 bedeckt und vorzugsweise mit den Außenwänden 2101a und 2101d fest verbunden. So bilden die Druckübertragungsmembranen einen Bestandteil der Außenwände des Gehäuses 2400. Außenwände 2101a und 2101d sind vorzugsweise gleich gestaltet und daher entspricht die Ansicht der Unterseite des Gehäuses 2400 ebenfalls der Darstellung in Figur 2a. Auch möglich ist eine Ausführungsform, bei der die Fenster 2102 und 2102' versetzt voneinander angeordnet sind, wodurch dann die Außenwände 2101a und 2101d sich durch die Position des Fensters unterscheiden.

Figur 2b zeigt Draufsichten A, B, und C der Ausführungsform der Drucksensorvorrichtung 2000 und deren Bestandteile. A in Figur 2b zeigt das Gehäusegerüst 2100 ohne MEMS-Druckchip und Druckübertragungsmembranen. Zu sehen ist die Außenwand 2101a des Gehäusegerüsts mit Fenster 2102, das Auflagemodul 2104 mit Verbindungsstelle 2108 sowie die Aussparungen in Form von Fugen 2106 und 2107. B in Figur 2b zeigt das Gehäusegerüst 2100 mit eingelegtem MEMS-Chip 1300. Dabei ist der MEMS-Chip 1300 so auf dem Auflagemodul gelagert, dass er durch die Fugen 2106 und 2107 an zwei Längsseiten vom Gehäusegerüst 2100 entkoppelt ist. C in Figur 2b zeigt das Gehäuse 2400 der Drucksensorvorrichtung, welches gebildet wird durch das Gehäusegerüst 2100 und den Druckübertragungsmembranen 2201 und 2202 (auf der Unterseite).

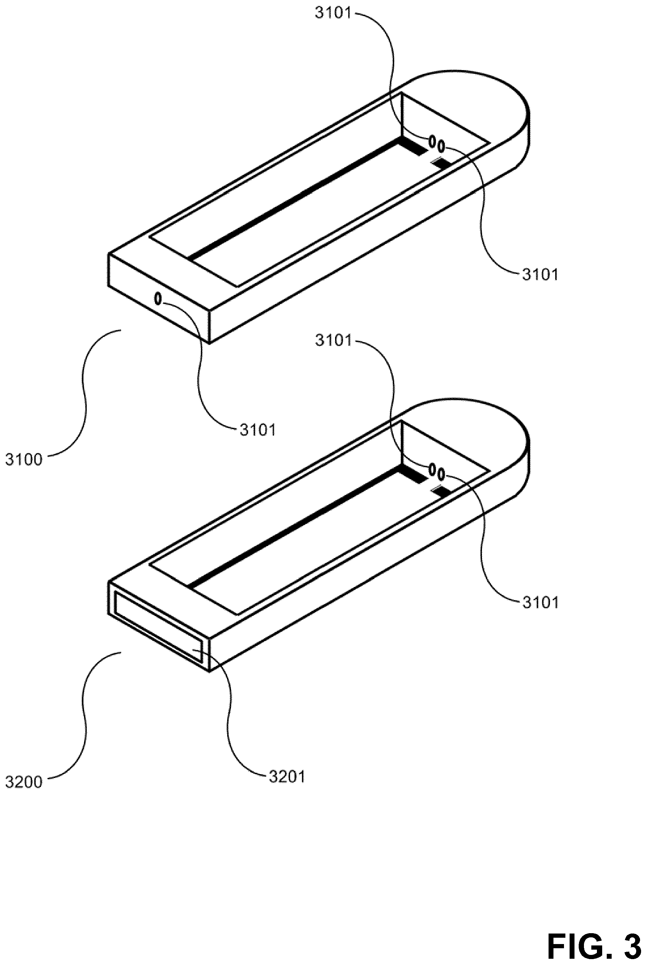

Figur 3 zeigt gemäß weiteren Ausführungsformen die Gehäuseanordnung mit Durchbrüchen. Vorzugsweise sind die Durchbrüche am Gehäusegerüst vorzunehmen, sodass sie beispielhaft am Gehäusegerüst 2100 entsprechend Figur 2 dargestellt sind. Die Durchbrüche können jedoch auch für andere Formen des Gehäusegerüsts bzw. an anderer Stelle der Gehäuseanordnung vorgenommen werden. Das Gehäusegerüst 3100 besitzt Durchbrüche in Form von kleinen Löchern 3101, welche an den Stirnseiten platziert sind. Gehäusegerüst 3200 besitzt an einer Stirnseite einen Durchbruch in rechteckiger Form 3201. Die Durchbrüche können beispielsweise als Durchführungen für elektrische Kontakte genutzt werden, beispielsweise um den im Inneren des Gehäuses liegenden MEMS-Chip mit anderen elektrischen Komponenten zu verbinden. Auch können die Durchbrücke zum Befüllen des Innenvolumens der Gehäuseanordnung nach dem Zusammenbau mit dem Druckübertragungsmedium, z. B: Öl, genutzt werden. Für eine solche Nutzung des Durchbruchs ist dieser vorzugsweise verschließbar gestaltet.

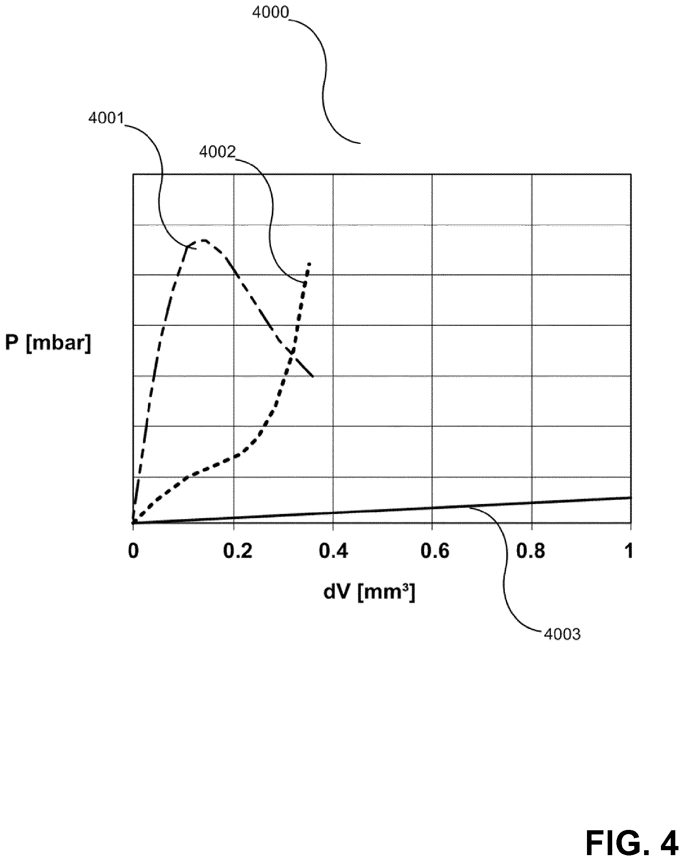

Figur 4 zeigt ein Druck-Volumen-Diagramm 4000 mit Druck-Volumen-Kurven von Drucksensorvorrichtungen mit verschiedenartigen Gehäuseanordnungen. Kurven 4001 und 4002 stellen Druckverhältnisse P in Abhängigkeit zur Volumenänderung ΔV im Inneren des Gehäuses für bekannte Gehäuseanordnungen dar, bei welchen eine Druckübertragungsmembran an einer Seite des Gehäuses montiert ist. Kurve 4003 repräsentiert das Druck-Volumen-Verhalten der Gehäuseanordnung. Es ist zu sehen, dass der Druck P der Kurven für 4001 und 4002 der bekannten Gehäuseanordnungen steil und nicht linear für bereits kleine Volumenänderungen ΔV verläuft, während der Druck P für die Gehäuseanordnung linear und auch bei großen Volumenänderungen ΔV nur geringfügig ansteigt.

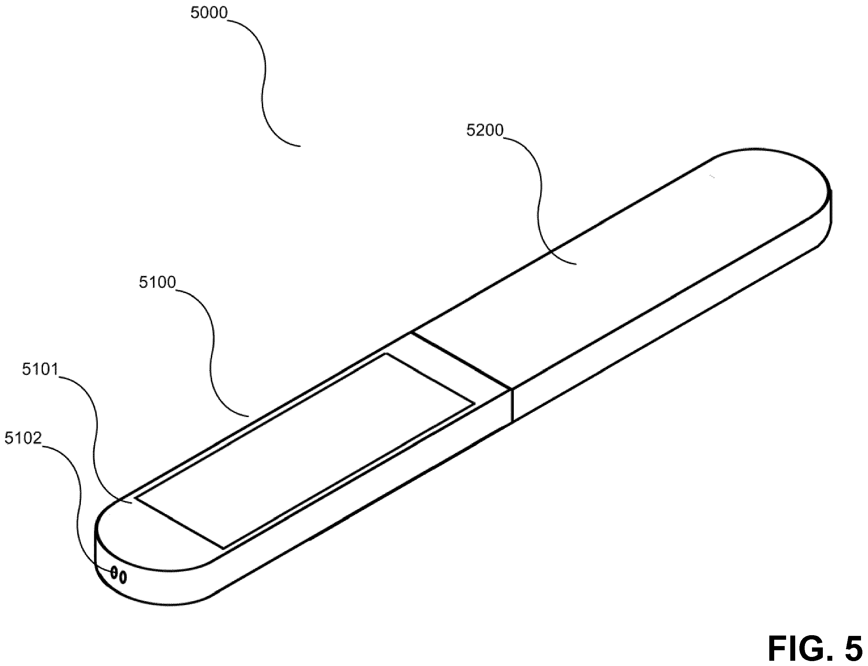

Figur 5 illustriert schematisch einen Aufbau 5000 bestehend aus einer Ausführung der implantierbaren Drucksensorvorrichtung 5100 und einem Modul 5200. Dargestellt ist die Drucksensorvorrichtung 5100 mit Gehäuse 5101, wobei das Gehäuse 5101 wahlweise Durchbrüche 5102 an den Stirnseiten aufweist. Das Modul 5200 ist fest mit der Drucksensorvorrichtung 5100 an einer flachen Stirnseite beider Komponenten verbunden. Dabei verfügen sowohl die Drucksensorvorrichtung 5100 als auch das Modul 5200 ein abgerundetes Ende, sodass der Aufbau 5000 über keine Ecken verfügt, was Vorteile für den Implantationsvorgang und die Biokompatibilität erzielt. Modul 5200 kann beispielsweise als Batteriemodul ausgestaltet sein oder andere elektronische Komponenten beherbergen, wie z.B. Datenspeichereinheiten oder eine Recheneinheit für die drahtlose Kommunikation. Drucksensorvorrichtung 5100 und Modul 5200 können beispielsweise über einen Durchbruch an den Kontaktstellen beider Module elektrisch verbunden werden, wie in Figur 3 beispielhaft dargestellt.

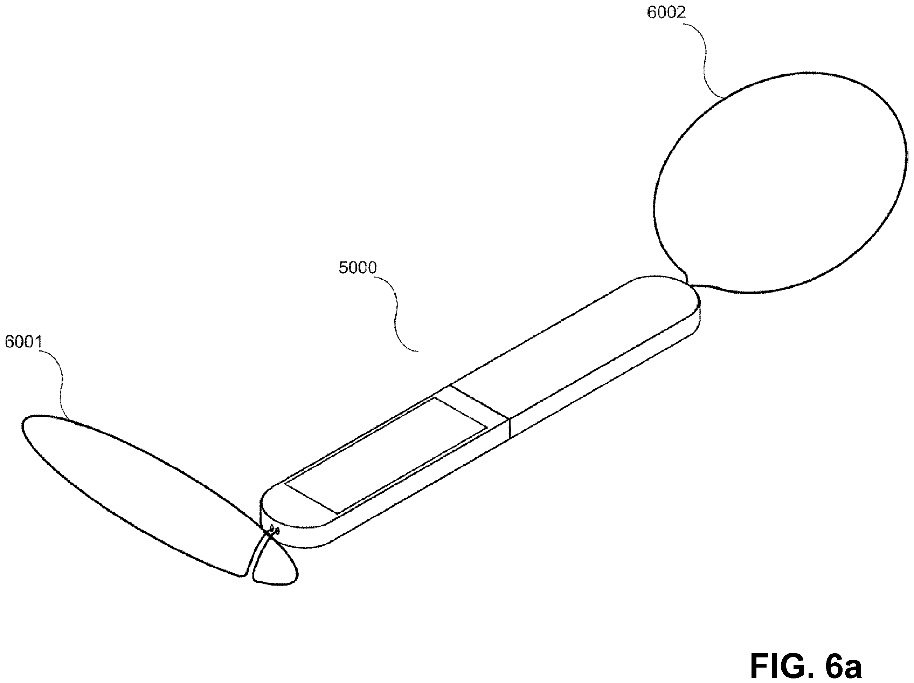

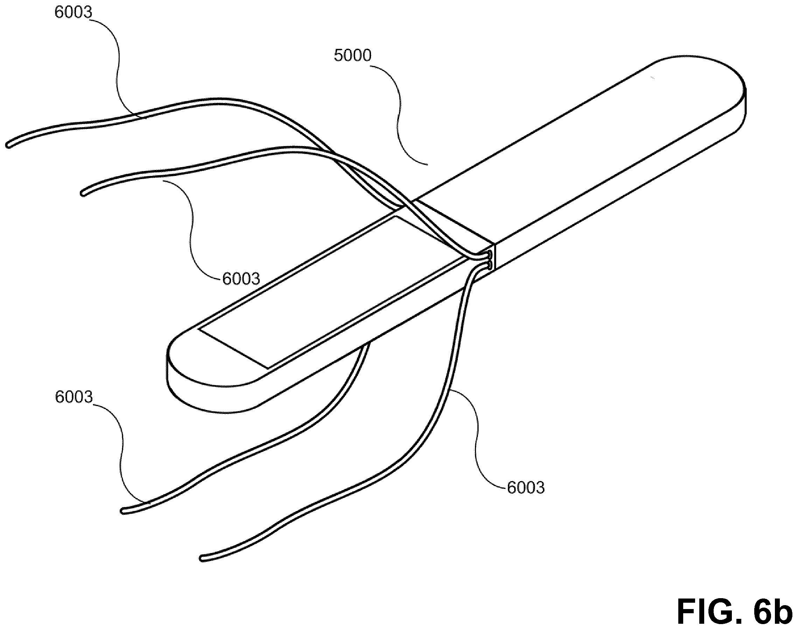

Figuren 6a und 6b zeigen schematisch mögliche Konstruktionen für Aufbau 5000 aus Figur 5, mit denen dieser am Implantationsort stabilisiert bzw. verankert werden kann.

Figur 6a zeigt Aufbau 5000 mit an den Stirnseiten befestigten Drahtschlaufen 6001 und 6002. Die Drahtschlaufen können Aufbau 5000 an einem Implantationsort halten, der einen größeren Durchmesser besitzt als Aufbau 5000 selbst, wie zum Beispiel für einen Einsatz in der Pulmonararterie. Gleichzeitig können Körperflüssigkeiten, wie Blut, weiter durchströmen. Des Weiteren können Drahtschlaufen 6001 und/oder 6002 wahlweise als Antenne für eine drahtlose Kommunikation der Drucksensorvorrichtung mit einem externen Gerät genutzt werden. Vorteilhafterweise sind Drahtschlaufen 6001 und 6002 vor und während des Implantationsvorgangs in einem zusammengefalteten Zustand, sodass die Beförderung zum Implantationsort erleichtert wird, und können am Implantationsort in einen entfalteten Zustand gebracht werden. Auch kann der Aufbau 5000 mit der in Figur 6b abgebildeten Lösung am Implantationsort gehalten werden. Bei dieser Lösung sind Drahtarme 6003 an Aufbau 5000 befestigt, die ebenfalls die Befestigung des Implantats an einem Implantationsort gewährleisten, der einen größeren Durchmesser besitzt als Aufbau 5000 selbst. Dabei können die Drahtarme 6003 an Öffnungen angebracht sein, die am Gehäuse der Drucksensorvorrichtung angebracht sind. Bevorzugt sind die Drahtarme elastisch ausgestaltet.

### Bezugszeichenliste

- 1000: Implantierbare Drucksensorvorrichtung
- 1100: Gehäusegerüst
- 1101a-1101d: Außenwände
- 1102: Fenster in Außenwand 1101a
- 1102': Fenster in Außenwand 1101d
- 1103: Innenvolumen
- 1104: Auflagemodul
- 1105: Vorsprung
- 1106, 1107: Fugen
- 1108: Verbindungsstelle
- 1201, 1202: Druckübertragungsmembranen
- 1300: MEMS-Druckchip
- 1400: Gehäuse
- 1500: Weiteres Gehäusegerüst ohne Vorsprung
- 1501: Auflagemodul für weiteres Gehäusegerüst
- 1502: Verbindungsstelle für weiteres Gehäusegerüst
- 2000: Weitere implantierbare Drucksensorvorrichtung (w.i.D.)
- 2100: Gehäusegerüst der w.i.D.
- 2101a-2101d: Außenwänder der w.i.D.
- 2102: Fenster in 2101a
- 2102': Fenster in 2101d
- 2103: Innenvolumen der w.i.D.
- 2104: Auflagemodul der w.i.D.
- 2106, 2107: Fugen der w.i.D.
- 2108: Verbindungsstelle der w.i.D.
- 2201, 2202: Druckübertragungsmembranen der w.i.D.
- 2400: Gehäuse der w.i.D.
- 3100: Gehäusegerüst mit Durchbrüchen
- 3200: Weiteres Gehäusegerüst mit Durchbrüchen
- 4000: PV Diagramm
- 4001, 4002: PV Kurven von bekannten Gehäuseanordnungen
- 4003: PV Kurve der offenbarten Gehäuseanordnung
- 5000: Aufbau :Drucksensorvorrichtung mit angeschlossenem Modul
- 5100: Drucksensorvorrichtung
- 5101: Gehäuse
- 5102: Durchbrüche
- 5200: Modul
- 6001, 6002: Drahtschlaufen
- 6003: Drahtarme

## Patentansprüche

1. Implantierbare Drucksensorvorrichtung (1000; 2000) mit einer Gehäuseanordnung, wobei
- die Gehäuseanordnung Außenwände (1101a - 1101d; 2101a - 2101d) und ein Innenvolumen (1103; 2103)) aufweist,
- in dem Innenvolumen (1103; 2103) ein mikroelektromechanischer Drucksensorchip (1300) auf einem Auflagemodul (1104; 2104) wie auf einem Steg gelagert ist, und
- die Gehäuseanordnung zumindest zwei Druckübertragungsmembranen (1201, 1202; 2201, 2202) aufweist, wobei die Druckübertragungsmembranen (1201, 1202; 2201, 2202) jeweils eine Oberfläche besitzen und die Oberflächen nicht in einer Ebene angeordnet sind, wobei
- Fugen (1106, 1107; 2106, 2107) entlang der Längsseiten des Auflagemoduls (1104; 2104) verlaufen, derart, dass das Auflagemodul (1104; 2104) ebendort von den Außenwänden (1101a - 1101d; 2101a - 2101d) entkoppelt ist, und
- eine Verbindungsstelle (1108; 2108) zwischen Enden der Fugen (1106, 1107; 2106, 2106) eine Materialverbindung zwischen dem Auflagemodul (1104; 2104) und der Gehäuseanordnung bildet.

2. Vorrichtung nach Anspruch 1, wobei die Druckübertragungsmembranen (1201, 1202; 2201, 2202) einen Bestandteil von mindestens zwei unterschiedlichen Außenwänden bilden.

3. Vorrichtung nach Anspruch 2, wobei die zwei unterschiedlichen Außenwände sich gegenüberliegen.

4. Vorrichtung nach einem der vorigen Ansprüche, wobei mindestens eine Außenwand der Gehäuseanordnung einen reversibel deformierbaren Bereich aufweist.

5. Vorrichtung nach einem der Ansprüche 1 bis 4, wobei mindestens eine Druckübertragungsmembran (1201, 1202; 2201, 2202) an die Fugen (1104; 2104) grenzt oder gekoppelt ist.

6. Vorrichtung nach einem der Ansprüche 1 bis 5, wobei mindestens eine Außenwand an die Fugen (1104; 2104)grenzt oder gekoppelt ist.

7. Vorrichtung nach einem der vorigen Ansprüche, wobei die Gehäuseanordnung zumindest teilweise als Quader, Zylinder, Kugel oder Ellipsoid gestaltet ist.

8. Vorrichtung nach einem der vorigen Ansprüche, wobei das Innenvolumen (1103; 2103) der Gehäuseanordnung mit einem Druckübertragungsmedium befüllbar ist.

9. Vorrichtung nach einem der vorigen Ansprüche, wobei die Außenwände (1101a - 1101d; 2101a - 2101d) der Gehäuseanordnung mindestens einen Durchbruch zum Innenvolumen (1103; 2103) der Gehäuseanordnung aufweisen.

## Claims

1. An implantable pressure sensor device (1000; 2000) having a housing arrangement, wherein
- the housing arrangement has outer walls (1101a-1101d; 2101a-2101d) and an internal volume (1103; 2103),
- in the internal volume (1103; 2103) a microelectromechanical pressure sensor chip (1300) is mounted on a support module (1104; 2104), as on a rib, and
- the housing arrangement has at least two pressure transfer membranes (1201, 1202; 2201, 2202), wherein the pressure transfer membranes (1201, 1202; 2201, 2202) each have a surface and the surfaces are not arranged in one plane,
wherein
- joints (1106, 1107; 2106, 2107) extend along the longitudinal sides of the support module (1104; 2104) in such a way that the support module (1104; 2104) is decoupled there from the outer walls (1101a-1101d; 2101a-2101d), and
- a connection point (1108; 2108) between ends of the joints (1106, 1107; 2107, 2106) forms a material connection between the support module (1104; 2104) and the housing arrangement.

2. The device according to claim 1, wherein the pressure transfer membranes (1201, 1202; 2201, 2202) form a part of at least two different outer walls.

3. The device according to claim 2, wherein the two different outer walls are opposite each other.

4. The device according to any one of the preceding claims, wherein at least one outer wall of the housing arrangement has a reversibly deformable region.

5. The device according to any one of claims 1 to 4, wherein at least one pressure transfer membrane (1201, 1202; 2201, 2202) borders or is coupled to the joints (1104; 2104).

6. The device according to any one of claims 1 to 5, wherein at least one outer wall borders or is coupled to the joints (1104; 2104).

7. The device according to any one of the preceding claims, wherein the housing arrangement is formed at least in part as a cuboid, cylinder, sphere or ellipsoid.

8. The device according to any one of the preceding claims, wherein the internal volume (1103; 2103) of the housing arrangement can be filled with a pressure transfer medium.

9. The device according to any one of the preceding claims, wherein the outer walls (1101a-1101d; 2101a-2101d) of the housing arrangement have at least one aperture to the internal volume (1103; 2103) of the housing arrangement.

## Revendications

1. Dispositif de capteur de pression implantable (1000; 2000) doté d'un agencement de boîtier, dans lequel
- l'agencement de boîtier présente des parois extérieures (1101a à 1101d ; 2101a à 2101d) et un volume intérieur (1103 ; 2103),
- une puce de capteur de pression (1300) micro électromécanique est logée dans le volume intérieur (1103 ; 2103) comme posée sur une tige, et
- l'agencement de boîtier présente au moins deux membranes de transmission de pression (1201, 1202 ; 2201, 2202), les membranes de transmission de pression (1201, 1202 ; 2201, 2202) possédant respectivement une surface et les surfaces n'étant pas disposées dans un plan,
où
- des joints (1106, 1107 ; 2106, 2107) s'étendent sur le long des côtés longitudinaux du module d'appui (1104 ; 2104) de telle manière que le module d'appui (1104 ; 2104) est désaccouplé des parois extérieures (1101a à 1101d ; 2101a à 2101d) juste à ces endroits là, et
- un point de liaison (1108 ; 2108) entre des extrémités des joints (1106, 1107 ; 2106, 2106) forme une liaison à base de matière entre le module d'appui (1104 ; 2104) de l'agencement de boîtier.

2. Dispositif selon la revendication 1, dans lequel les membranes de transmission de pression (1201, 1202 ; 2201, 2202) forment un composant à base d'au moins deux parois extérieures différentes.

3. Dispositif selon la revendication 2, dans lequel les deux parois extérieures différentes sont situées l'une en face de l'autre.

4. Dispositif selon l'une des revendications précédentes, dans lequel au moins une paroi extérieure de l'agencement de boîtier présente une région déformable de manière réversible.

5. Dispositif selon l'une des revendications 1 à 4, dans lequel au moins une membrane de transmission de pression (1201, 1202 ; 2201, 2202) est limitrophe ou est couplée avec les joints (1104 ; 2104).

6. Dispositif selon l'une des revendications 1 à 5, dans lequel au moins une paroi extérieure est limitrophe ou est couplée avec les joints (1104 ; 2104).

7. Dispositif selon l'une des revendications précédentes, dans lequel l'agencement de boîtier est conçu au moins partiellement sous forme d'un parallélépipède rectangle, d'un cylindre, d'une sphère ou d'un ellipsoïde.

8. Dispositif selon l'une des revendications précédentes, dans lequel le volume intérieur (1103 ; 2103) de l'agencement de boîtier peut être rempli avec un milieu de transmission de la pression.

9. Dispositif selon l'une des revendications précédentes, dans lequel les parois extérieures (1101a à 1101d ; 2101a à 2101d) de l'agencement de boîtier présentent au moins une ouverture vers le volume intérieur (1103 ; 2103) de l'agencement de boîtier.
